# EUROPEAN PATENT APPLICATION

(11) **EP 3 117 799 A1**
(43) Date of publication of application: **18.01.2017**
(21) Application number: 16179313.8
(22) Date of filing: 13.07.2016
(51) Int. Cl.: A61F 2/04, A61F 2/848

(54) **STENT ANTI-MIGRATION MECHANISM**

(30) Priority: 15.07.2015 US 201562192791 P
(71) Applicant: Cook Medical Technologies LLC, Bloomington, IN 47404 (US)
(72) Inventor: RYAN, Michael, Limerick (IE); HOULIHAN, Gerard, Co Limerick (IE)
(74) Representative: Garratt, Peter Douglas

(57) **Abstract**

A prosthesis and a method for implantation of the prosthesis into a bodily lumen are provided. The prosthesis includes a body having a proximal portion, a distal portion, an outer surface and a lumen extending therethrough defining an inner surface. The outer surface includes a first plurality of protrusions, the first plurality of protrusions are adapted to increase the friction force between the prosthesis and the bodily lumen, the first plurality of protrusions forming a first pattern on at least a portion of the outer surface of the body.

## Description

### RELATED APPLICATIONS

This application claims the benefit of U.S. Provisional Application No. 62/192,791, filed July 15, 2015, which is incorporated by reference herein in its entirety.

### FIELD OF THE INVENTION

The present invention relates to medical devices and in particular to a prosthesis having an anti-migration mechanism.

### BACKGROUND OF THE INVENTION

Stent migration is a known complication associated with the use of self-expanding metal stents (SEMS). Stent migration can occur immediately after stent placement or at some delayed time after placement of the stent. The gastrointestinal tract is a very dynamic environment due to the mechanisms that enable food transportation, i.e., motility. In the esophagus for example, the waves of peristaltic motion pass along the esophagus. The amplitude of these waves varies along the length of the esophagus and from patient to patient. Both the esophageal diameter and length change with the peristaltic motion of the esophagus.

In addition to the environment for stent placement being dynamic, the stent itself may be dynamic. In the case of foreshortening stents, e.g. EVOLUTION stents (Cook Medical Inc., Bloomington, IN), the stent length changes as the stent diameter changes. Esophageal motility may cause the stent to creep along the esophageal lumen.

Attempts have been made to address stent migration using different modifications that have varying results. One example is a partially covered stent. The benefit of a partially covered stent is that the partially covered stent facilitates tissue ingrowth around the uncovered portion of the stent and enabling stent anchorage. However, the tissue ingrowth occurs at a delayed time after stent placement. Initially, the partially covered stent is susceptible to stent migration and once the ingrowth does occur, stent removal becomes more challenging.

The use of removable stents is increasing, such as for benign strictures or neoadjuvant therapy. What is needed in the art is a stent that may be removable yet resistant to migration within a dynamic implant site.

### BRIEF SUMMARY

Accordingly, it is an object of the present invention to provide a device and a method having features that resolve or improve on the above-described drawbacks.

In one aspect, a prosthesis for implantation into a bodily lumen is provided. The prosthesis includes a body having a proximal portion, a distal portion, an outer surface and a lumen extending therethrough defining an inner surface. The outer surface includes a first plurality of protrusions, the first plurality of protrusions are adapted to increase the friction force between the prosthesis and the bodily lumen, the first plurality of protrusions forming a first pattern on at least a portion of the outer surface of the body. The body may comprise an expandable structure with a liquid impermeable sleeve surrounding the expandable structure and the protrusions may protrude from an otherwise smooth external surface of the sleeve. The body may comprise an expandable structure formed of a first material and the protrusions may be formed of a second, different material. The protrusions may be formed in a molding process. The protrusions may be formed of polymeric material.

In another aspect, a method of anchoring a prosthesis in a bodily lumen is provided. The method includes inserting a prosthesis into a bodily lumen in a collapsed configuration and positioning the prosthesis in the bodily lumen and expanding the prosthesis so that a first plurality of protrusions on an outer surface of a body of the prosthesis formed in a first pattern on the outer surface contact a wall of the bodily lumen and a frictional force is created between the first plurality of protrusions and the wall.

### BRIEF DESCRIPTION OF THE DRAWINGS

FIG. 1 is a partial side view of an embodiment of a prosthetic device in accordance with the present invention;
FIG. 2 is a partial side view an embodiment of a prosthetic device showing a pattern of protrusions;
FIG. 3 is a sectional view of the embodiment of the prosthetic device shown in FIG. 1;
FIGS. 4A-4F illustrate cross-sectional profiles of embodiments of the protrusions of the prosthetic device;
FIGS. 5A-5C illustrate cross-sectional profiles of embodiments of the protrusions of the prosthetic device;
FIGS. 5D-5F illustrate an exemplary interaction of the prosthetic device shown in FIG. 5A with a tissue;
FIGS. 6A-6I illustrate cross-sectional profiles of embodiments of the protrusions of the prosthetic device;
FIG. 7A-7E illustrate partial views of exemplary patterns for the protrusions;
FIGS. 8A-8J illustrate exemplary patterns of protrusions on the outer surface of the prosthesis;
FIGS. 9A-9B illustrate a w pattern of protrusions that extends over the weave of the prosthesis;
FIGS. 10A-10B illustrate an x pattern of protrusions that extends over the weave of the prosthesis;
FIGS. 11A-11B illustrate a step pattern of protrusions that extends over the weave of the prosthesis;
FIGS. 12A-12B illustrate a step pattern of protrusions that extends over the weave of the prosthesis;
FIGS. 13A-13E illustrate examples of patterns of protrusions that may be included on portions of the prosthesis;
FIGS. 14A-14B illustrate an embodiment of the prosthesis having protrusions angled toward a distal end;
FIG. 15 illustrates an embodiment of a mold cavity;
FIG. 16A illustrates an embodiment of the prosthesis positioned in a bodily lumen;
FIGS. 16B-16C show an enlarged view of a portion of the prosthesis and a wall of the bodily lumen; and
FIGS. 17A-17B illustrate the interaction of the prosthesis with the bodily lumen in an expanded configuration and a compressed configuration.

### DETAILED DESCRIPTION

The invention is described with reference to the drawings in which like elements are referred to by like numerals. The relationship and functioning of the various elements of this invention are better understood by the following detailed description. However, the embodiments of this invention are not limited to the embodiments illustrated in the drawings. It should be understood that the drawings are not to scale, and in certain instances details have been omitted which are not necessary for an understanding of the present invention, such as conventional fabrication and assembly.

As used in the specification, the terms proximal and distal should be understood as being in the terms of a physician delivering the prosthesis to a patient. Hence the term "distal" means the portion of the prosthesis that is farthest from the physician and the term "proximal" means the portion of the prosthesis that is nearest to the physician.

The present invention relates to medical devices, and in particular to prosthetic devices for implantation in a body lumen such as the esophagus or a vessel. As used herein, the term "implantable" refers to an ability of a medical device to be positioned at a location within a body, such as within a body lumen, either temporarily, semi-permanently, or permanently. Permanent fixation of the device in a particular position is not required. Furthermore, the terms "implantation" and "implanted" refer to the positioning of a medical device at a location within a body, such as within a body lumen. In some instances, the implanted device may be removed after a period of time.

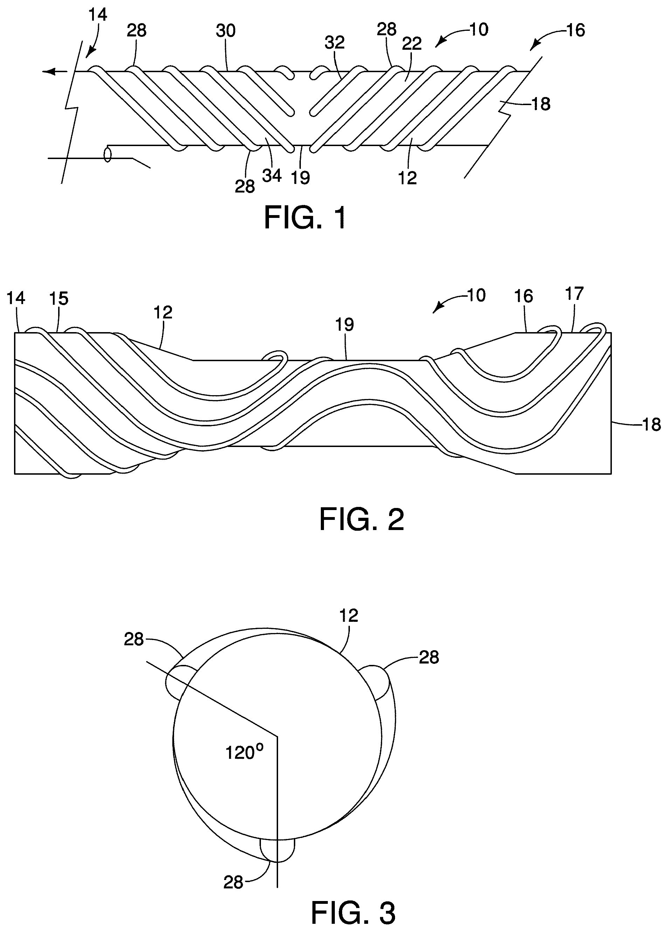

FIG. 1 illustrates a prosthesis 10 in accordance with an embodiment of the present invention. The prosthesis 10 includes a body 12 having proximal portion 14, a distal portion 16 and a lumen 18 extending therethrough. In some embodiments, the body 12 may be an expandable stent, such as a self-expanding stent. Non-limiting examples of expandable stents include the Z-Stent^{®} and the EVOLUTION^{®} stent (Cook Medical, Inc., Bloomington, IN). In some embodiments, the body 12 may be a non-expandable tubular stent. The prosthesis 10 may be an esophageal, duodenal, colonic, biliary or other type of stent. In some embodiments, the proximal portion 14 may include an end portion 15 having an expanded outer diameter and the distal portion 16 may include an end portion 17 having an expanded outer diameter relative to a mid-portion 19 diameter. The body 12 may include a coating or a sleeve 22 extending through or around the body 12 or a portion of the body 12. The sleeve 22 may be substantially liquid impermeable and may provide a barrier or partial barrier to tissue ingrowth at the implant site.

The prosthesis 10 also includes one or more protrusions 28 on an outer surface 30 of the body 12 that extend outward from the outer surface 30. The protrusions 28 cover a portion 32 of the outer surface 30 and a portion 34 of the outer surface remains free of protrusions 28. The protrusions 28 inhibit stent migration by increasing the friction force of the prosthesis 10 against the wall of a body lumen into which the prosthesis 10 is implanted. The protrusions 28 are adapted to act cooperatively with each other to provide a cumulative overall friction to resist movement of the body 12 within the bodily lumen. The protrusions 28 are sized and shaped so as not to be so large or sharp that will the protrusions 28 will cause a tear or injury to the bodily lumen. In some embodiments, the protrusions 28 may be micro or nano protrusions. By way of non-limiting example, micro protrusions may have a height up to about 0.5 mm and in some embodiment a height up to about 0.3 mm. In some embodiments, the micro protrusions may have a diameter of up to about 0.2 mm. By way of non-limiting example, non-protrusions may have a height up to about 0.3 mm and a diameter of up to about 0.1 mm. Larger protrusions may also be used. For example, in some embodiments, the height may be up to about 3 mm, about 2 mm or about 1.5 mm. In some embodiments the diameter of the protrusions may be up to about 1 mm. In some embodiments including a wire, the protrusions may be have a diameter not greater than the diameter of the wire. The protrusions 28 are adapted to facilitate immediate anchorage of the prosthesis within the body and also allow for removal of the prosthesis at a later time. The protrusions 28 may be provided in many configurations as described in more detail below.

FIGS. 1-3 illustrate an embodiment of the protrusions 28 extending from the outer surface 30. As shown, the protrusions 28 may be provided as one or more helical patterns that extend from the mid portion 19 outwards towards the proximal portion 14 and the distal portion 16. FIG. 3 shows three helical protrusions 38 that are spaced apart around a circumference of the body 12 by about 120°. The helical protrusions 28 may form a continuous raised helix on the outer surface 30 as shown in FIGS. 1 and 2. Alternatively, the helical protrusions 28 may be made up of a plurality of raised protrusions 28 that together form a helix or other pattern as described below. Other patterns and spacing are also possible for the protrusions 28.

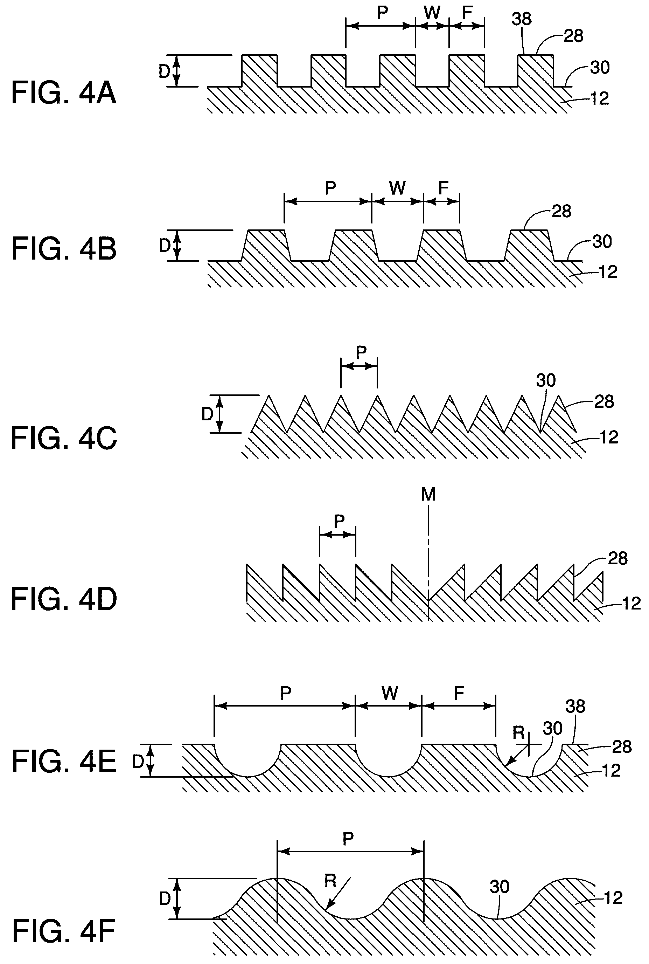

FIGS. 4A-4F illustrate non-limiting examples of cross-sectional views of profile configurations for the protrusions 28 that may be used to form a pattern on the outer surface 30 of the body 12 and may be used with any of the embodiments of the prosthesis described herein. FIG. 4A illustrates an embodiment where the protrusions 28 have a square profile. Each protrusion 28 may extend a distance D away from the outer surface 30 of the body 12. A peak distance P measured between adjacent peaks of the square profile protrusions 28 and a width W between adjacent protrusions 28 that corresponds to the outer surface 30 of the body 12 is shown in FIG. 4A. A face F of the protrusion 28 extends across an outer surface 38 of the protrusion 28. By way of non-limiting example, the square profile protrusions 28 may be used to form different patterns on the outer surface 30 of the body 12 such as one or more vertical, horizontal, double helical, woven, diamond or screw-thread patterns. For each of the profile configurations for the protrusions described in this application, one or more of the distance D, the peak distance P, the width W and length of the face F may be optimized for the implant site, the prosthesis type and length to facilitate anchorage of the prosthesis at the implant site. In some embodiments, the distance D may be varied within a profile configuration so that the distance D is not the same for each protrusion 28. Similarly, the peak distance P, the width W and length of the face F may be varied within a profile configuration.

FIG. 4B illustrates an embodiment where the protrusions 28 have a trapezoidal profile. Similar to the square profile protrusion 28 shown described above, each protrusion 28 may extend a distance D away from the outer surface 30 of the body 12. A peak distance P measured between adjacent peaks of the trapezoidal profile protrusions 28 and a width W between adjacent protrusions 28. A face F of the protrusion 28 extends across an outer surface 38 of the protrusion 28. By way of non-limiting example, the trapezoidal profile protrusions 28 may be used to form different patterns on the outer surface 30 of the body 12 such as one or more vertical, horizontal, diamond or screw-thread patterns.

FIG. 4C illustrates an embodiment where the protrusions 28 have a v-shaped profile. Each protrusion 28 may extend a distance D away from the outer surface 30 of the body 12. A peak distance P measured between adjacent peaks of the v-shaped profile protrusions 28. The base of the v corresponds to the outer surface 30 of the body 12. In some embodiments, the v-shaped profile protrusions 28 may be used to form different patterns on the outer surface 30 of the body 12 such as one or more screw-thread patterns.

FIG. 4D illustrates an embodiment where the protrusions 28 have a double saw edge profile. Each protrusion 28 may extend a distance D away from the outer surface 30 of the body 12. A peak distance P measured between adjacent peaks of the double saw edge profile protrusions 28. As shown in FIG. 4D, a midpoint M may define a change in the angular extension of the saw edge so that a portion of the protrusions 28 are angled in a first direction and a portion of the protrusions 28 are angled in a second direction. In some embodiments, the double saw edge profile protrusions 28 may be used to form different patterns on the outer surface 30 of the body 12 such as one or more double helical patterns.

FIG. 4E illustrates an embodiment where the protrusions 28 have a half circle profile. Each protrusion 28 may extend a distance D away from the outer surface 30 of the body 12. A peak distance P measured between adjacent peaks of the half circle profile protrusions 28 and a width W between adjacent protrusions 28. A face F of the protrusion 28 extends across an outer surface 38 of the protrusion 28. In some embodiments, the length of the face F may be a point between adjacent half circles. (Not shown.) A radius R of the half circle is shown in FIG. 4E. The length of the radius R may be optimized for the implant site, the prosthesis type and length to facilitate anchorage of the prosthesis at the implant site. The radius R may be varied within a profile so that not all the protrusions have the same radius R. In some embodiments, the half circle profile protrusions 28 may be used to form different patterns on the outer surface 30 of the body 12 such as one or more vertical or screw-thread patterns.

FIG. 4F illustrates an embodiment where the protrusions 28 have a rounded profile. Each protrusion 28 may extend a distance D away from the outer surface 30 of the body 12. A peak distance P measured between adjacent peaks of the rounded profile protrusions 28. A radius R of the rounded profile is shown in FIG. 4F. The length of the radius R may be optimized for the implant site, the prosthesis type and length to facilitate anchorage of the prosthesis at the implant site. In some embodiments, the rounded profile protrusions 28 may be used to form different patterns on the outer surface 30 of the body 12 such as one or more vertical, horizontal or screw-thread patterns.

In some embodiments, the distance D may be up to about 10 mm, the width W may be up to about 5 mm, length of the face F may be up to about 0.5 mm. In some embodiments, shorter lengths may be used for one or more of the lengths described. In some embodiments longer lengths may be used for one or more of the lengths described.

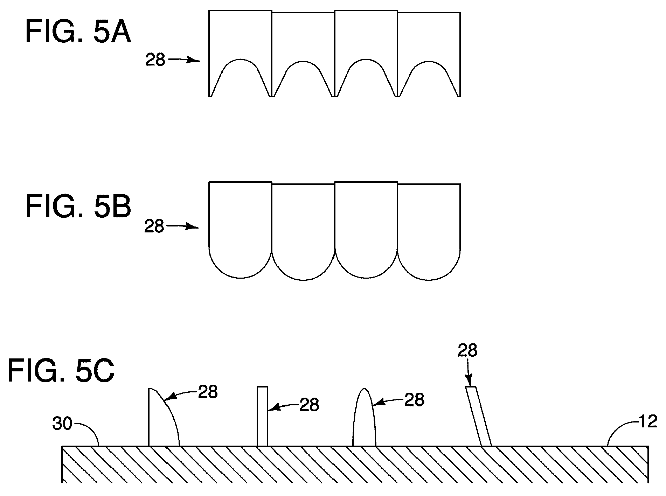

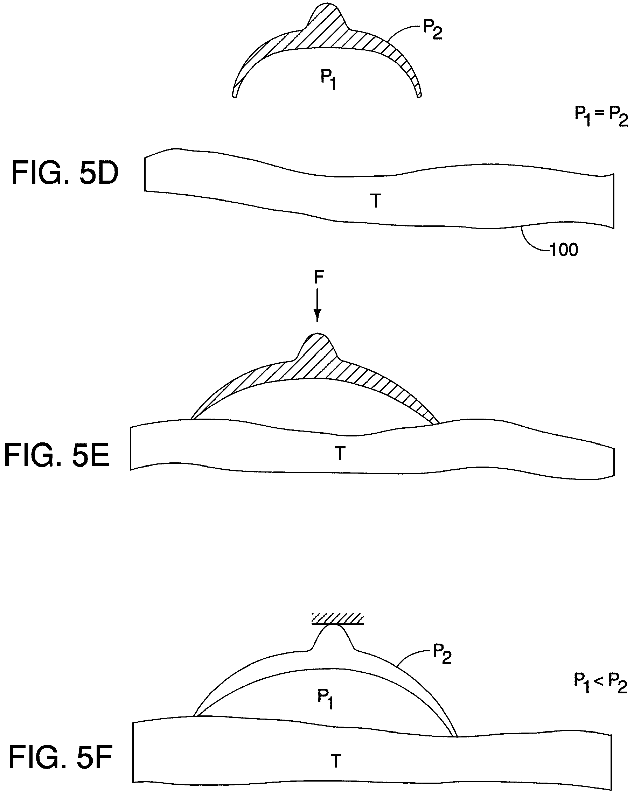

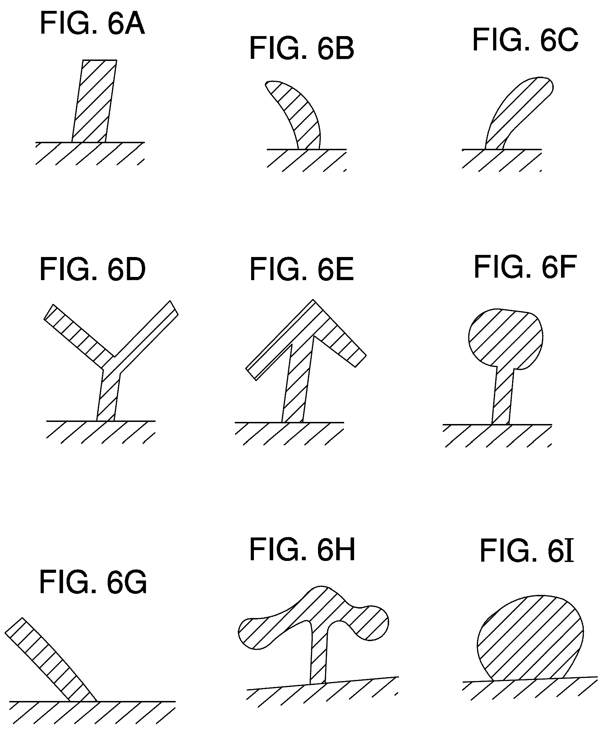

FIGS. 5A-5C and 6A-6I illustrate additional cross-sectional views of profile configurations for the profiles 28 that may be used with any of the embodiments of the prosthesis described herein. In the embodiment shown in FIG. 5A, the plurality of protrusions 28 may be sized and shaped to act like suction cups that anchor the prosthesis 10 to the wall of the bodily lumen when the prosthesis is implanted. By way of non-limiting example, the protrusions 28 in the form of suction cups contact the tissue and as the stent self expands, the air is removed from the cups causing the suctions cups to grip to the tissue. FIGS. 5D-5F illustrate a partial view the interaction of the prosthesis 12 with a tissue T when the protrusions 28 form suction cups as shown in FIG. 5A. FIG. 5D illustrates the prosthesis 12 spaced apart from the tissue T where pressures P1 and P2 on either side of the prosthesis 12 are equal. FIG. 5E illustrates a force F exerted on the prosthesis 12 pressing the prosthesis 12 against the tissue T and FIG. 5F illustrates the prosthesis 12 interacting with the tissue T where P1 is less than P2. FIG. 5B illustrates a plurality of rounded protrusions 28 that curve outward and may interact with the luminal wall at the implant site such as the gastrointestinal tract having an undulating surface on the mucosal layer.

FIGS. 5C and 6A-6I illustrate alternative exemplary cross-sectional shapes for the protrusions 28. The protrusions 28 may have the same cross-sectional shape or different cross-sectional shape covering a portion of the outer surface 30 of the body 12. The protrusions 28 may be positioned on the outer surface 30 in any of the patterns described herein. In some embodiments, such as shown in FIG. 5C, the protrusions 28 may extend away from the body 12 up to a height of about 1 mm. In some embodiments, the protrusions 28 extend less than a height of about 1 mm.

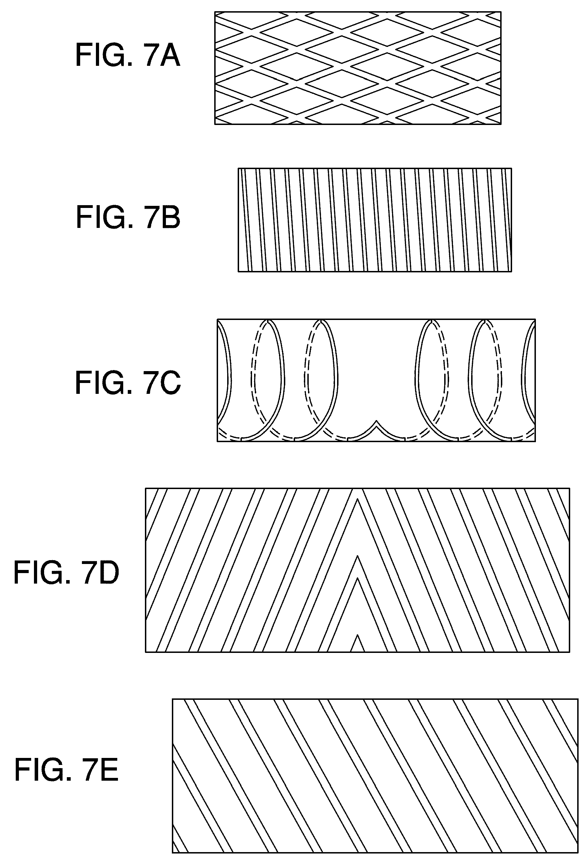

Exemplary patterns that may be formed using the any of the protrusions 28 described herein are shown for example in FIGS. 7-13. The pattern on the prosthesis may be formed so that air trapped between the mucosal layer and the outer surface 30 of the prosthesis 10 may be released to provide increased contact between the protrusions 28 and the mucosal surface of the bodily lumen at the implant site. In some embodiments, the pattern on the prosthesis may be formed so that there is resistance to peristaltic movement in one, two or more directions, for example in the antegrade and retrograde directions. The pattern may also be formed to resist rotational migration. In embodiments having one or more helical patterns, the helical pattern may be the same as the helical pitch of a woven or wire prosthesis, while other pitches for the pattern are also possible. The helical pitch of the pattern may be constant or variable.

The pattern of the protrusions 28 may be over the entire prosthesis 10 or portions thereof. In some embodiments, the pattern may be formed on the proximal portion 14 of the body 12, the distal portion 16 or both. In some embodiments the pattern of protrusions 28 may be formed on one or both end portions 15, 17 of the body 12 having an expanded outer diameter. In yet other embodiments, the pattern of protrusions 28 may be formed on the mid portion 19 of the body 12 only. In other embodiments, for example in esophageal embodiments, the pattern of protrusions 28 may be formed on the proximal portion 14 and/or end portion 15 only so that the protrusions 28 contact healthy tissue and are anchored thereto.

FIGS. 7A to 7E illustrate exemplary patterns that may be provided on the outer surface 30 of the body 12. FIG. 7A shows a diamond pattern, FIG. 7B shows a vertical pattern, FIG. 7C shows a worm pattern, FIG. 7D shows a double helical pattern and FIG. 7E shows a helical pattern. Each of the patterns may be formed with a plurality of protrusions 28 having any of the cross-sectional profiles described above.

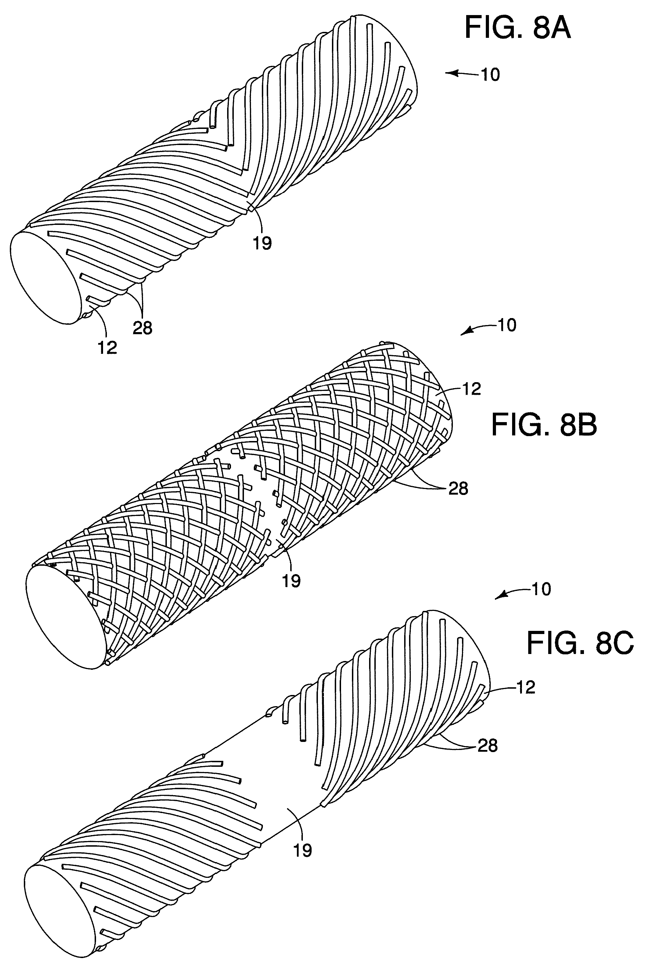

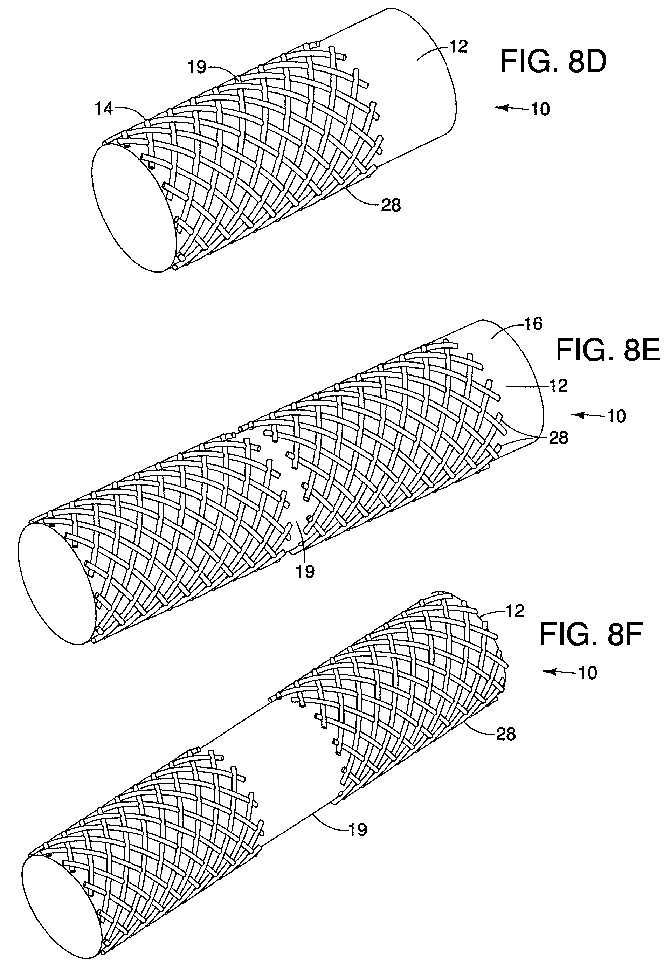

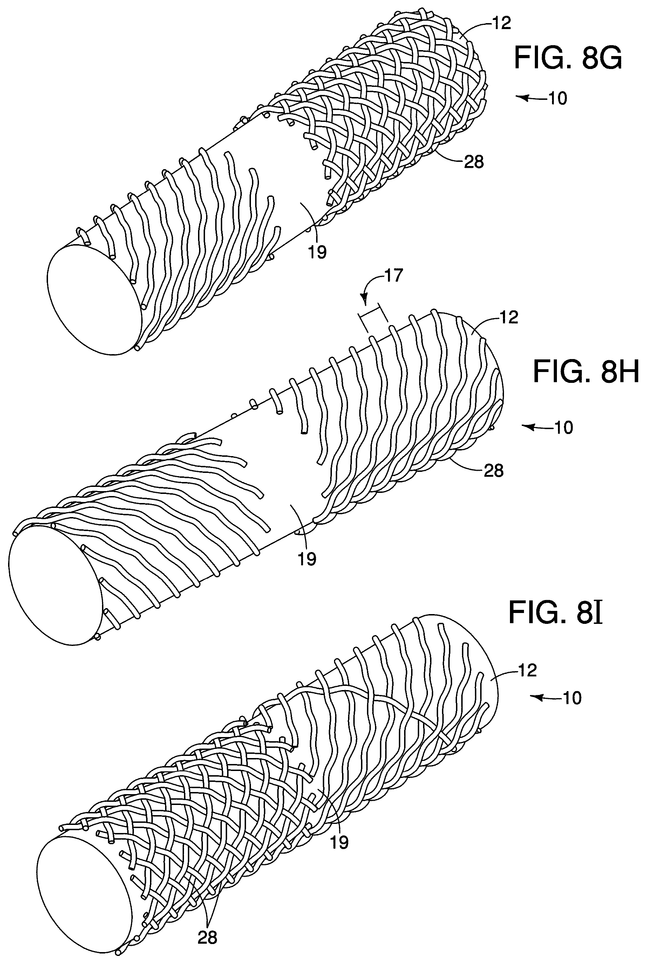

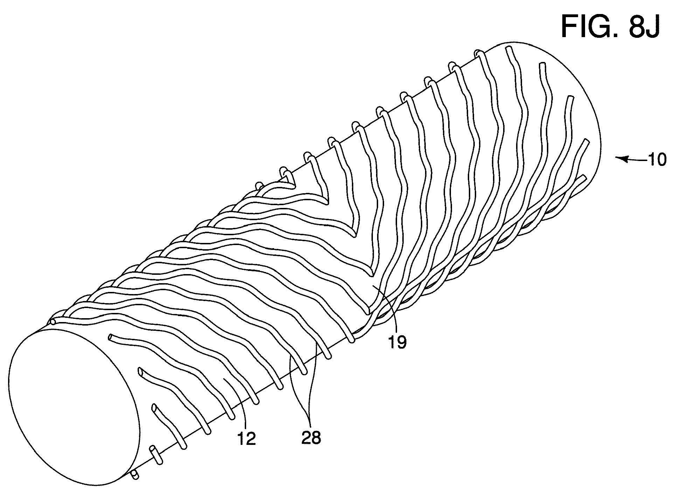

Figures 8A-8J illustrate exemplary patterns of protrusions 28 on the outer surface 30 of the prosthesis 10. As shown in FIGS. 8A, 8I and 8J, the pattern may extend over the body 12 including the entire mid portion 19 and FIGS. 8A and 8J show the patterns extending outward from the mid-portion 19 in opposite directions. In some embodiments, the mid portion 19 may be free of protrusions 28 as shown in FIGS. 8B, 8C, 8E, 8F, 8G, and 8H. The amount of protrusion free portions may be varied. FIGS. 8A-8C show examples of patterns that extend to ends of the body 12, while FIGS. 8D and 8E show examples of patterns where one or both ends of the body may be free of protrusions 28. FIG. 8D illustrates an example of a pattern extending across the mid portion 19 and a proximal portion 14 of the body 12. FIGS. 8B, 8D, 8E, 8F, 8G and 8I illustrate embodiments where at least a portion of the pattern is woven. FIGS. 8G and 8I illustrate examples of patterns where different patterns cover different portions of the body 12. In some embodiments, a wire may be used to form the protrusions 28. By way of non-limiting example, the wire may be less than about 0.5 mm and in some embodiments the wire may be less than about 0.3 mm in diameter. In some embodiments, a spacing 17 between adjacent wire segments may be up to about 5 mm. See for example, FIG. 8H.

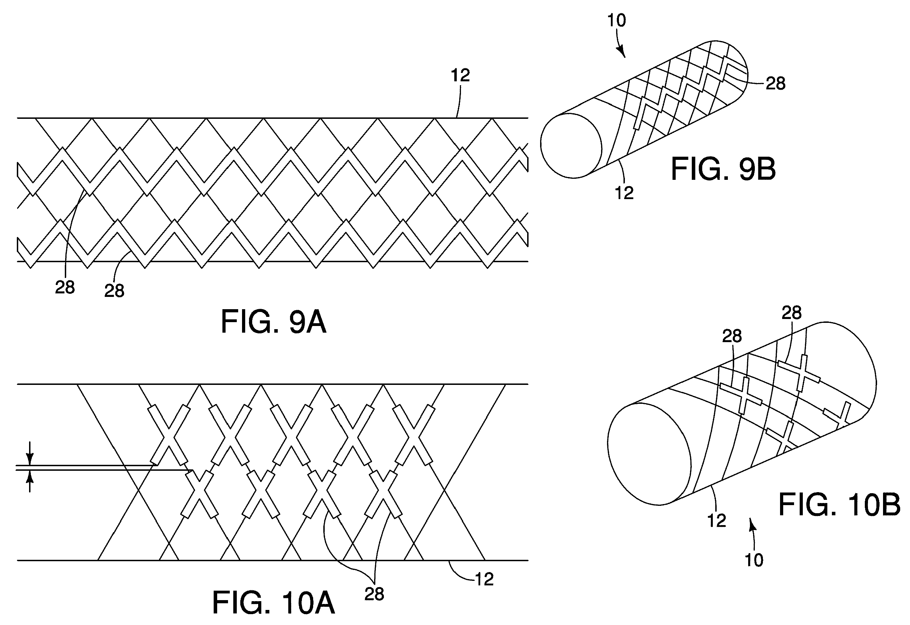

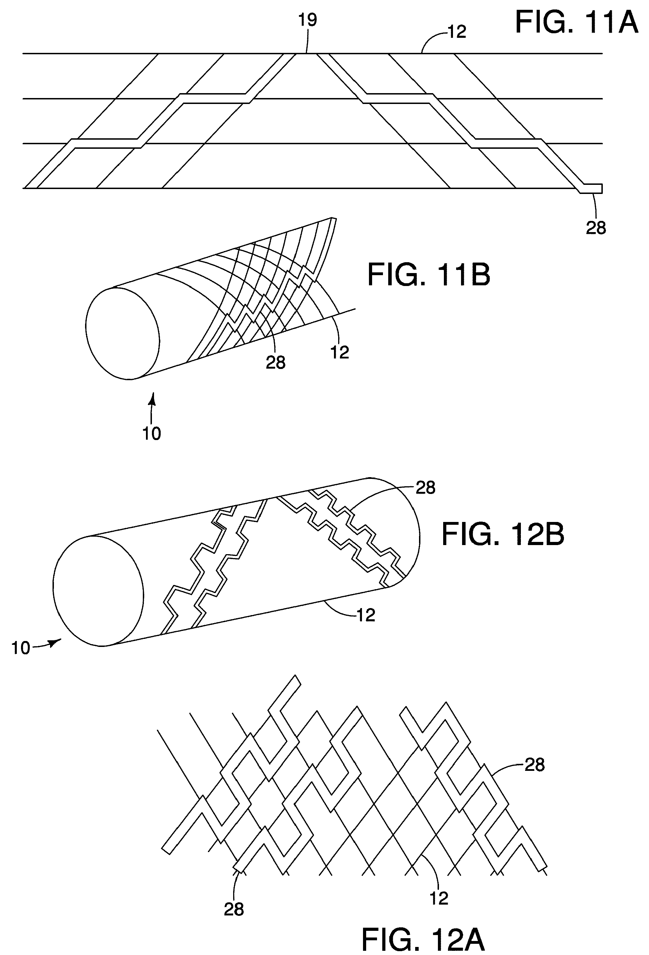

FIGS. 9-12 illustrate examples of patterns that may be used with woven prostheses where the pattern of protrusions 28 corresponds to a portion of the weave that forms the body 12 of the prosthesis 10. FIGS. 9A, 10A, 11A and 12A show an enlarged view of the pattern of protrusions 28 overlaying the pattern of the weave of the body 12. FIGS. 9B, 10B, 11B and 12B show the pattern of protrusions 28 on the prosthesis 10. FIGS. 9A and 9B illustrate a w pattern of protrusions 28 that extends over the weave of the prosthesis 10. The w pattern may include 1, 2, or more rows of w's extending the entire length of the prosthesis 10 or a portion thereof. FIGS. 10A and 10B illustrate an x pattern of protrusions 28 that extends over the weave of the prosthesis 10. The x pattern may include 1, 2, or x's extending over the entire length of the prosthesis 10 or a portion thereof. FIGS. 11A-11B and 12A-12B illustrate a step pattern of protrusions 28 that extends over the weave of the prosthesis 10. The step pattern may include 1, 2, or more rows of steps extending the entire length of the prosthesis 10 or a portion thereof and may extend around the circumference of the prosthesis 10 in a helical pattern. In some embodiments, two or more step patterns of protrusions 28 may be provided and extend in different directions away from the mid portion 19 of the prosthesis 10 in a helical or other pattern.

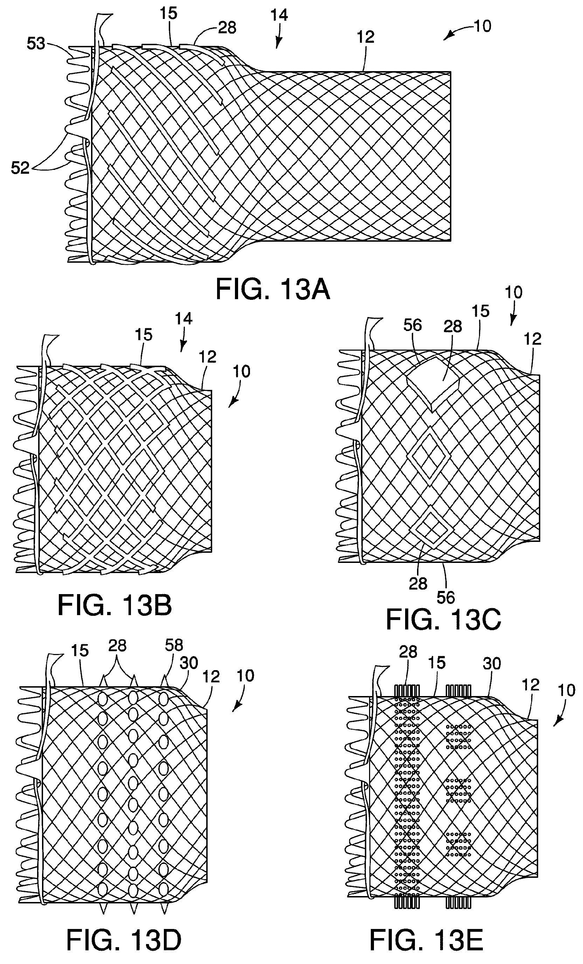

FIGS. 13A-13E illustrate examples of patterns that may be included with a prosthesis 10, where the pattern is shown on the end portion 15 having an expanded outer diameter. While the proximal portion 14 of the body 12 is shown with the end portion 15 having the expanded outer diameter, the pattern of protrusions 28 may be provided on the proximal portion 14, the distal portion 16 or both and with or without the expanded outer diameter. The reminder of the body 12 may be free of protrusions 28. As shown in FIG. 13A, the prosthesis 10 may be a self-expanding woven prosthesis having a suture 50 threaded through loops 52 to facilitate delivery of the prosthesis 10 using a delivery system such as described in US Publications US 20120041538 and 2011/0190865, but not limited thereto. Each of the embodiments described herein may be delivered using a delivery system. These embodiments are also removable due to the configurations of the protrusions 28. By way of non-limiting example, the prosthesis 10 have any pattern describe herein may be removed by collapsing the prosthesis such as by pulling on sutures/loops attached to the prosthesis to collapse the prosthesis. (See US Publication US 20120041538, for example.) Each end of the prosthesis may have the suture 50 and loops 52. The protrusions 28 are formed to be able to move with the prosthesis 10 from a collapsed configuration to an expanded configuration and back to a collapsed configuration if needed. FIG. 13A shows the pattern of protrusions 28 overlaying the pattern of the weave of the end portion 15 of the prosthesis 10. The pattern of the protrusions 28 may be formed to extend at an angle to a longitudinal axis of the prosthesis 10 without the protrusions 28 overlapping as shown in FIG. 13A or alternatively, the pattern of protrusions may overly the pattern of the weave of the prosthesis 10 and overlap, for example in a series of crisscrossing extensions as shown in FIG. 13B. In some embodiments the pattern of protrusions may overlay the pattern of the weave in a square or diamond shape as shown in FIG. 13C. An interior portion 56 of the square or diamond shaped pattern may be filled with protrusions 28, partially filled with protrusions 28 or free of protrusions 28. In some embodiments, the interior portion 56 may be filled or partially filled with a soft material that conforms to the shape of the bodily lumen at the implant site.

FIG. 13D illustrates an embodiment showing protrusions 28 that are prism or conically shaped with a prism or cone tip 58 pointing out from the outer surface 30 of the prosthesis 10. The protrusions 28 shown in FIG. 13D may be positioned in open cells of the woven prosthesis 10 or at intersections of the woven material. The protrusions 28 may be formed in one or more rows as shown in FIG. 13D, but other patterns are also possible. FIG. 13E illustrates an embodiment of the pattern of protrusions 28 on the end portion 15 of the prosthesis 10 having a plurality of protrusions 28 that are straight cylinders or rectangles extending outward from the outer surface 30 of the prosthesis 10. The protrusions 28 may be grouped in clusters, continuous or other configurations and arranged in rows or other configurations.

In some embodiments, the protrusions 28 on the end portion 15 may have a wall thickness of up to about 1 mm and in some embodiments up to about 0.5 mm or less.

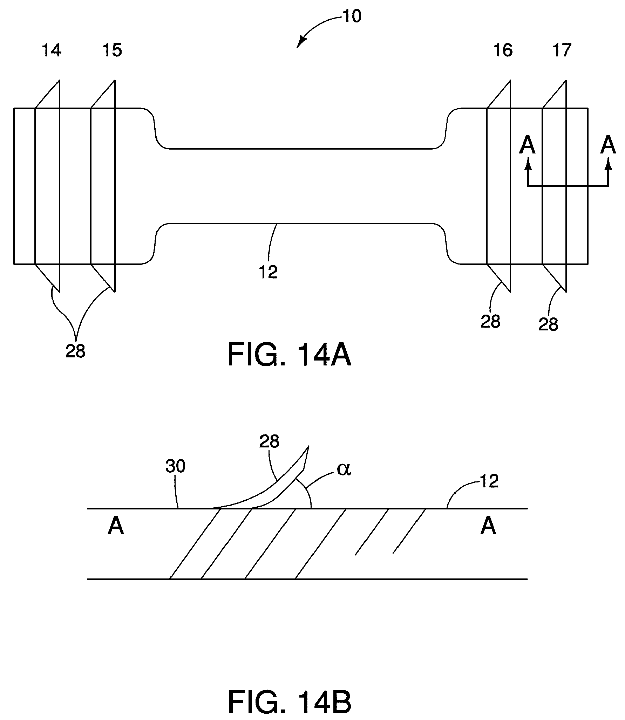

FIGS. 14A-14B illustrate an embodiment of the prosthesis 10 having a plurality of annular ring patterns of protrusions 28 on the proximal portion 14 and the distal portion 16. As shown in the sectional view, the protrusions 28 may be provided at an angle a extending away from the body 12 where the angle a is less than about 90°. The protrusions 28 may extend toward the distal portion 16 so that once the prosthesis 10 is implanted, the prosthesis is restricted from moving distally and allow the prosthesis 10 to be withdrawn proximally. The pattern of the protrusions 29 may be any of the patterns described above. By way of non-limiting example, the protrusions 28 may be angled toward the distal portion 16 and form a helical pattern around the prosthesis 10 or a portion thereof.

The materials used to manufacture the components of the prosthetic devices described herein may be any materials known to one skilled in the art that are suitable for use in patients. By way of non-limiting example, the body may be formed from metals or polymers. Suitable exemplary metals include stainless steel and nitinol and the body may be woven or provided in a zig-zag configuration. Portions of the prosthetic devices of the embodiments may be made from any suitable biocompatible material that does not degrade in the presence of fluids or gastric material that comes in contact therewith. By way of non-limiting example, the protrusions may be made from a medical grade polyurethane material, silicone, nylon, polyamides such as other urethanes, polyethylene , polyethylene terephthalate (PET), polystyrene-ethylene (PSE), polytetrafluoroethylene (PTFE), ultrahigh molecular weight, low density and high density polyethylene, elastomeric polyethylene, polyethyleneoxide (PEO), block copolymers containing polystyrene and poly(1,4-butadiene), ABA triblock copolymer made from poly(2-methyl-2-oxazoline), polytetrahydrofuran, shape memory polymers, amorphous or organic-inorganic hybrid polymers containing polymorbornere units or other biocompatible materials that are flexible and acid resistant. In some embodiments, portions of the prosthesis may be made from biodegradable materials such as, but not limited to polylactic acid (PLA), poly(lactic-co-glycolic acid) (PLGA), polyglycolic acid (PGA), PBA, polydioxanone, polyglactin, polyglyconate and poly-L-lactide (PLLA) and combinations thereof.

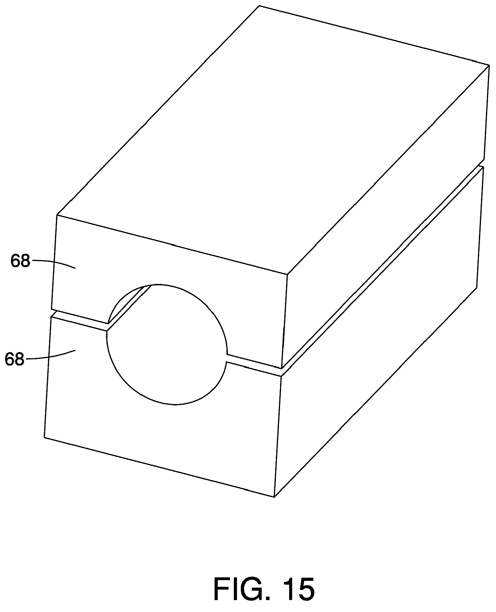

The body 12 of the prosthesis 10 may be manufactured using techniques known to one of skill in the art. The protrusions may be manufactured by molding, weaving, dipping, combinations thereof or by other methods. In some embodiments, the protrusions may be manufactured using an upper and lower mold cavity 66, 68 as shown in FIG. 15. The upper and lower mold cavities 66, 68 may be formed so that the mold cavities 66, 68 have indentations formed in the desired pattern for the protrusions 28 on the prosthesis 10. By way of non-limiting example, the protrusions may be formed from a silicone material that is added to the mold cavities 66, 68 with the body 12 and cured. In some embodiments, the cured material forming the protrusions may be removed from the mold and placed over the body or inside the body of the prosthesis and attached. In some embodiments, the protrusions may be cured together with the body so that the protrusions are added to the prosthesis and the completed prosthesis is removed. In some embodiments, the protrusions may be added on the outer surface of the prosthesis using a tool and a material such as silicone having a sufficiently low viscosity can be used to mold or dip the protrusions onto the prosthesis. The protrusions may be cured onto a previously formed sleeve and form a bond. The tool may be made from a very smooth, low friction material such as PTFE, glass, stainless steel or PEEK.

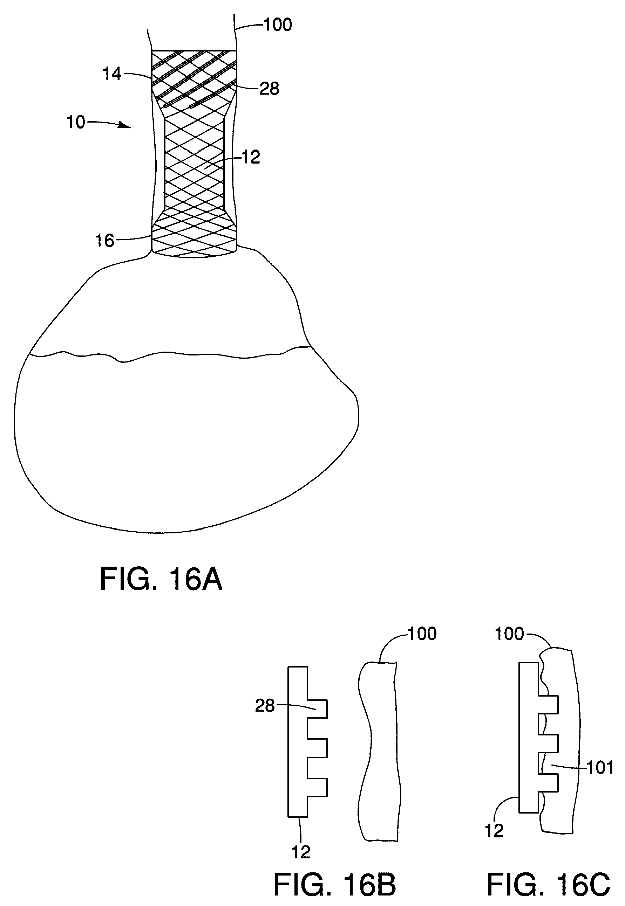

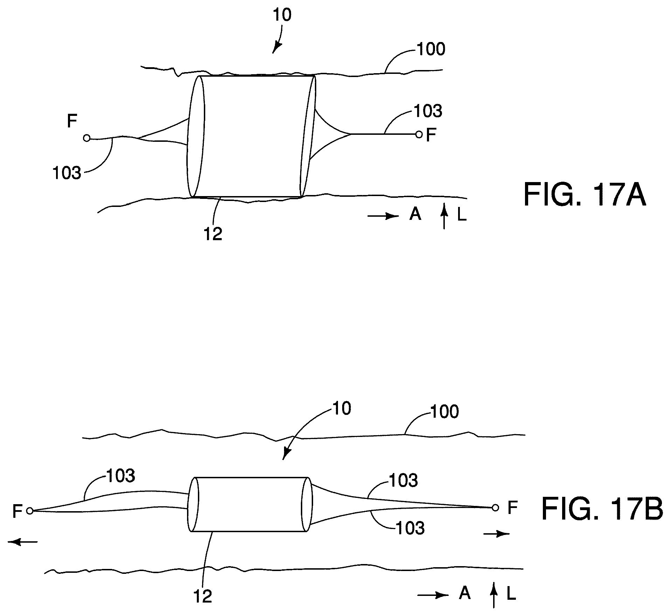

FIG. 16A illustrates an embodiment of the prosthesis 10 positioned within the esophagus 100. As shown, the proximal portion 14 of the body 12 includes the pattern of protrusions 28. The prosthesis 10 is in an expanded configuration after being delivered to the site in a collapsed configuration, for example as described in US20110190865 and as shown in FIGS. 17A and 17B. In the expanded configuration, the prosthesis 10 contacts a wall of the esophagus 100 and is anchored in the esophagus 100 via the frictional force created between protrusions/microvilli 101 on the wall of the esophagus 100 and the protrusions 28 of the body 12. FIG. 16B shows an enlarged view of the protrusions 28 of the body 12 spaced apart from the protrusions 101 of the wall 100. An enlarged view of an exemplary interaction between the protrusions 28 of the body 12 and protrusions 101 of the esophageal wall 100 is shown in FIG. 16C. The protrusions 28 inhibit migration of the prosthesis 10 by increasing the friction force of the prosthesis 10 against the wall of esophagus 100. The protrusions 28 are adapted to act cooperatively with each other and protrusions 101 of the wall 100 to provide a cumulative overall friction to resist movement of the body 12 within the implant site such as the esophagus. The embodiments of the prosthesis described herein may also be implanted at other sites within the body.

FIGS. 17A and 17B illustrate the expanded and compressed configurations of an exemplary prosthesis 10 within a bodily lumen, such as the esophagus 100. In the expanded configuration shown in FIG. 17A, the protrusions 28 of the body 12 are frictionally engaged with the protrusions 101 of the wall 100 as shown in FIG. 16C which prevents axial movement (A) but allows lateral movement (L). For example, as illustrated in FIG. 17B, when a force (F) is applied to the body 12, such as by pulling on sutures 103 connected to the body 12 in opposite directions, the body 12 is compressed and the interaction of the protrusions 28 of the body 12 with the protrusions 101 of the wall 100 is released by lateral movement of the protrusions 28 of the body 12 and the protrusions 101 of the wall 100 relative to each other to release the friction and allow the body 12 to be removed from the site or repositioned. FIG. 16B illustrates the relationship of the protrusions 28 of the body 12 and the protrusions 101 of the wall 100 when the prosthesis 10 is moved to the compressed configuration.

The above Figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. A prosthesis for implantation into a bodily lumen, the prosthesis comprising:
a body having a proximal portion, a distal portion, an outer surface and a lumen extending therethrough defining an inner surface, the outer surface comprising a first plurality of protrusions, the first plurality of protrusions increasing a contact surface area of the prosthesis and being adapted to increase the friction force between the prosthesis and the bodily lumen, the first plurality of protrusions forming a first pattern on at least a portion of the outer surface.

2. The prosthesis according to claim 1, wherein the prosthesis is self-expanding.

3. The prosthesis according to claim 1 or 2, wherein the prosthesis comprises a woven pattern.

4. The prosthesis according to claim 3, wherein the first plurality of protrusions overlay at least a portion of the woven pattern of the prosthesis.

5. The prosthesis according to any one of claims 1-3, wherein the first plurality of protrusions extend through openings formed in the body.

6. The prosthesis according to claim 3, wherein the first plurality of protrusions overlay intersection of the woven pattern.

7. The prosthesis according to any one of claims 1-6, wherein the first plurality of protrusions are formed on the proximal portion, the distal portion of the body or both the proximal portion and the distal portion.

8. The prosthesis according to any one of claims 1-7 wherein the prosthesis comprises a second plurality of protrusions having a second pattern.

9. The prosthesis according to any one of claims 1-8, wherein a mid-portion of the body is free of any protrusions.

10. The prosthesis according to claim 8, wherein the first and second patterns of protrusions meet at a mid-portion of the body.

11. The prosthesis according to any one of claims 1-10, wherein the first plurality of protrusions have a plurality of shapes.

12. The prosthesis according to any one of claims 1-11, wherein the first plurality of protrusions extend at an angle away from the body and towards a distal end of the body.

13. The prosthesis according to any one of claims 1-12, wherein the first pattern and/or the second pattern comprises a helical pattern.

14. The prosthesis according to any one of claims 1-12, wherein the first pattern comprises a crisscross pattern.

15. The prosthesis according to any one of claims 1-14 wherein a cross-sectional profile of one or more of the first plurality of protrusions comprises a square profile, a trapezoid profile, a v-profile, a double saw edge profile, a half circle profile or a round profile.
